# EUROPEAN PATENT APPLICATION

(11) **EP 2 689 793 A1**
(43) Date of publication of application: **29.01.2014**
(21) Application number: 12178390.6
(22) Date of filing: 27.07.2012
(51) Int. Cl.: A61M 5/24, A61M 5/20

(54) **Injection device for aesthetic medicine**

(71) Applicant: juvaplus SA, 1211 Geneve 12 (CH)
(72) Inventor: Konandreas, Stefanos, 1209 Petit-Saconnex (CH); Legrand, Bernard, 1231 Conches (CH)
(74) Representative: Sammer, Thomas

(57) **Abstract**

The present invention concerns an injection device (10) for aesthetic medicine, for administering a filling material beneath the skin of a person by means of a syringe (2). The injection device (10) comprises a motor system (4) with a motor (4.1) configured to drive a first piston (3.1), and at least a syringe cover (1,1') adapted to be mounted on the motor system (4) and to accommodate a syringe (2) comprising at least a syringe barrel (2.4) for accommodating the filling material, a syringe needle (2.1) and a second piston (2.2) located in the syringe and drivable by the first piston (3.1) for ejecting a quantity of filling material out of the syringe (2). The motor system (4) is cable-free electrically actuated.

## Description

The present invention pertains to an injection device for aesthetic medicine, for administering a viscous material beneath the skin of a person by means of a syringe. The injection device comprises a motor system with a motor configured to drive a first piston, and at least a syringe cover adapted to be mounted on the motor system and to accommodate a syringe comprising at least a syringe barrel for accommodating the injection material, a syringe needle and a second piston located in the syringe and drivable by the first piston for ejecting a quantity of injection material out of the syringe

In general, the present invention is situated in the context of techniques for aesthetic medicine, particularly for improving the external perception of a person's skin. A significant part of efforts in aesthetic medicine is directed to so called face injections. In that context, it is to be noted that there exist filler materials as well as botulinum toxin, often called by the brand name "Botox". Fillers are supposed to fill the underneath of a wrinkle that pre-exists on the patient's skin without impairing the patient's facial expression, i.e. his ability to move his face muscles. Botulinum toxin de-stimulates the muscles with the result that the person is unable to use a certain muscle group, e.g. while smiling, such as the frontalis muscles on the forehead. The manner in which fillers and botulinum toxin are injected is different due to the volume injected per patient, the viscosity of the fluids, and the way the fluid is deposited underneath the skin. The present invention is focused on providing a device for injection of both dermal fillers and botulinum toxin or similar products.

In particular, botulinum toxin shows a low viscosity, like water. Only small amounts have to be administered in order to de-stimulate a patient's specific muscle. Due to the low volume of botulinum toxin required for an injection, many doctors and practitioners use insulin syringes for the intervention. Typically, very thin syringes are used for botulinum toxin interventions, these syringes being standard syringes with a sharp tip. Challenges in the course of an injection of botulinum toxin are to avoid an application of too much fluid and to deposit the fluid exactly at the designated spot in order to avoid any damage to the patient.

In contrast to botulinum toxin, dermal fillers are very viscous liquids, comparable to hand lotion or thick soap. Face filling involves filling the skin with a "filler" material or filler liquid. Fillers are substances that adhere to the skin respectively the fat of the patient in order to create volume underneath the skin. This technique can be used for a number of reasons including but not limited to, hiding wrinkles by pushing the wrinkled skin out, giving volume to the lips, correcting the angle and shape of the nose, and filling up the upper cheeks.

For dermal fillers, typical quantities to be applied per injection are higher than for application of botulinum toxin. There are several types of filler materials, and several recipes of the same family. There are some older fillers such as collagen that are less used nowadays, the patient's own fat which may be extracted in a number of different ways, the most common filler material applied today being hyaluronic acid. There are several suppliers of hyaluronic acid, and each supplier has a portfolio of different filler materials as well as of syringe sizes, shapes and volumes, dedicated for filling different sections of the skin, e.g. deep wrinkles, superficial wrinkles, creating facial volume, etc.. Hyaluronic acid liquid is typically sold in a pre-filled syringe.

There are many different types of needles used for application of dermal fillers, respectively for injection of botulinum toxin or similar products, these being in the further course of the description globally designated as injection materials, since there are several techniques for skin treatment. Dermal fillers are generally more challenging to be handled than botulinum toxin in terms of the pressure to be applied due to its high viscosity, whereas injection of botulinum toxin is generally difficult because of the small quantities that have to be injected at specific locations. Hyaluronic acid injection does not only involve inserting and injecting a pre-defined, small quantity of fluid but injecting the needle under the entire length of the wrinkle and extract the syringe needle while injecting the injection material.

The needles used for filling operations vary in size, i.e. gauge and length, flexibility, and tip shape. The selection of the syringe depends on the kind of intended filling operation and the personal preference of the person performing the operation. Face filling requires a variable amount of force to be exerted on the syringe plunger, corresponding approx. to a weight in the range of 1 kg to 5 kg, in order to allow the viscous filler material to pass through the thin needle. Nevertheless, while the operation is being performed, a high level of manual control has to be guaranteed in order to place the right amount of fluid at the right spot. Therefore, it is particularly disadvantageous that - as generally known - the manual dexterity decreases when high forces have to be exerted. In several occasions it would be desirable to use thinner and longer syringe needles to perform the operation. This, however, is associated with the drawback that higher forces are needed to inject the fluid beneath the patient's skin. Suboptimal needle selection can particularly be disadvantageous in case of inability of the doctor or practitioner to deliver enough force to the needle in order to deliver the filling material. Filling the skin with filler material too fast or filling too much fluid in a dedicated area can result in unsatisfactory results and customer pain. This operation requiring a significant degree of dexterity is not made any easier by the fact that the doctor or practitioner has to press the plunger with high force while gently extracting the syringe from the patent and monitor the amount of fluid being injected. Another limiting factor for doctors and practitioners is the fact that the distance between the hand holding the syringe is far from where the syringe is being inserted. In order to sense and guide the syringe, the practitioner often uses his other hand to sense where the syringe is located.

The problems associated with using a syringe for delivering injection material are a recognized issue. A few companies have proposed solutions including electro-mechanical systems, pneumatic devices and/or simple mechanical dispensing aides as described further below. Although the injecting aid mechanisms that have been developed effectively eliminate or reduce the need for doctors and practitioners to press on the plunger, their weight, size, and complexity has resulted in them not being adopted by the mass market. The most common system used up to now are still standard syringes equipped with syringe plunger aides that simply distribute the force applied to a larger finger surface area of the middle, index and thumb.

In the following, some explicit examples of prior art injection devices will be discussed, mainly concerning devices adapted for injection of dermal fillers. U.S. patent application No. 2010/0105003 (Primequal/Switzerland) discloses a hand-piece, disposable device for ejecting a liquid or pasty product, comprising a body that has a portion designed to contain the product to be ejected and that has a hole for ejecting the product, a rack that moves in a lumen in the body and causes the volume of the portion designed to contain the product to vary, and a rack movement mechanism comprising a pivoting lever and a pawl for acting on the rack. The pawl and the lever form a one-piece plastics structure. Ejections are effected manually by activating, i.e. pressing the lever, without a motorized support for reducing forces to be applied by an operator. Advantages of this device are its simple construction and low weight, together with a single positive ergonomic aspect of taking advantage of the dead space inside the palm of an operator's hand. Disadvantageously, particularly because of lack of motorized support, the application of this device is limited to ejections of low-viscous fluids like Botox, but it is not adequate for ejection of highly viscous media to be applied as dermal fillers. This device only delivers single-sized drops and is thus very limited.

In U.S. patent application No. 2009/0093787 (Medicis Pharmaceutical Corp./U.S.A.) is disclosed a syringe, particularly dedicated for administration of dermal fillersdermal fillers in cosmetic application, the syringe comprising a barrel portion, a cushion being positioned against said barrel portion. The cushion is configured to provide cushioning when said barrel portion is grasped by two adjacent fingers of a same hand and a plunger portion comprising a distal end adapted for insertion into a proximal end of said barrel portion. In some disclosed embodiments, the barrel cushion comprises flanges with an increased surface in order to reduce the forces per pressing area required to operate the syringe. The device is intended for improved ease of use for an operator during injections, aspirations, or the like, and is provided as hand-piece with an integrated syringe. However, also this device suffers from only manual operation, requiring a force of about 0.7 Nm for administration of a filler material like Restylane^{®}. Again, this product only delivers a single sized drop.

Anteis S.A. (Switzerland) has commercialized an injection device comprising an electric motor for driving a threaded screw acting as a plunger for driving a syringe piston for injection of a liquid from a pre-filled syringe, a hand-piece holder, a control unit connected to the motor, a foot pedal connected to the control unit, a power cable, and spare disposable parts for holding the needle. For use of the injection device, a pre-filled syringe is placed into a disposable, transparent needle holder, like is visible e.g. in U.S. Design patent No. D650,075 S. The needle holder is screwed into place onto the motor device. On a control base a program for driving the syringe / needle is selected before the needle is inserted into the patient for penetrating the skin. By means of the foot pedal, the device is activated for performing the injection. It is an advantage of this injection device, besides replacing manually driven by motorized injection, that the needle holder appears to be ergonomic, allowing an operator to see the syringe inside, together with an actually contained amount of liquid from a lateral, perpendicular perspective of view onto the needle holder, while simultaneously disguising the syringe to the patient. Disadvantageously, the system comprises a plurality of cable connections that restrict particularly the flexibility of the operator's, i.e. the doctor's movements during use, and a foot pedal for injection activation which prevents the operator from walking around the patient for changing his position during an injection. Additionally, activation by means of a foot pedal has to be regarded as a rather imprecise activation method. The installation of the device, consisting of six separate, discrete units, appears to be relatively complex and may have to be performed by the manufacturer or distributor at the application site. Furthermore, a syringe once inserted into the needle holder can then no longer be removed and has to be disposed together with the needle holder. As a further disadvantage, the needle holder is designed to house only a single type of syringes compatible to the ones provided by Anteis.

In the international patent application WO 2011/119811 (Nordson Corporation/U.S.A.) is disclosed a gas-assisted fluid-dispensing device for delivering an aerosol. The gas-assisted fluid-dispensing device includes a syringe that contains a fluid and includes a distal end, a proximal end, and a plunger extending proximally towards the distal end. A spray nozzle tip is configured to generate the aerosol and is coupled to the distal end of the syringe. The gas-assisted fluid-dispensing device further includes a housing having first and second housing portions. The first housing portion includes a docking port for receiving the syringe; the second housing portion is configured as a handle. A trigger, coupled to the housing, is connected to an actuating member that is configured to apply a force onto the plunger of the syringe and discharge the fluid from the syringe.

A related injection device product distributed by Nordson & Micromedics comprises a control unit with a hand-piece holder for a hand-piece to be applied by an operator, a foot pedal for activation of pneumatically assisted dispensing of fluids, and a syringe holder provided or providable with different adapters for accommodating syringes of different type and sizes. Advantageously, this system has already successfully been approved not only in aesthetic medicine, but also in other technical areas of application, for dispensing grease, glues and other fluids. From the user's perspective, applicability of many different types of syringes is also beneficial. However, also this system suffers from similar disadvantages as described above with respect to the Anteis system, namely the existence of several cables and wires and operation by using a foot pedal, which may be both imprecise and uncomfortable for an operator for the same reasons as set out above. Furthermore, pneumatically actuated activation generally appears to be a rather imprecise activation method.

The solutions according to prior art therefore all suffer from a lack of comfort of operating the injection devices, although the motor-assisted application of the filling material proposed by some known devices removes the requirement of applying strong forces for administering the injection from the operator.

It is the object of the present invention to overcome the above mentioned difficulties and to realize an injection device which is adapted both for injection of dermal fillers and for injection of botulinum toxin whilst providing more flexibility of use for an operator, a precise control of the device respectively of the quantity of injection material ejected at the desired location, as well as multiple ways of control, but simultaneously not requiring the exertion of significant forces for application of an injection. Also, the device should be adapted to be used with a wide variety pre-filled syringes originating from several manufacturers and the syringe should be less visible to the patient, for psychological reasons during performing the aesthetic intervention.

To this effect, the present invention proposes an injection device, which is characterized by the features enumerated in claim 1 and which allows to achieve the objectives identified above.

The problems associated with known injection devices as described above are solved, according to the invention, by an injection device for aesthetic medicine, for administering a filling material beneath the skin of a person by means of a syringe. The injection device comprises a motor system with a motor configured to drive a first piston, and at least a syringe cover adapted to be mounted on the motor system and to accommodate a syringe comprising at least a syringe barrel for housing the filling material, a syringe needle and a second piston located in the syringe and drivable by the first piston for ejecting a quantity of filling material out of the syringe. In particular, the injection device according to the present invention is characterized by the fact that the motor system is cable-free electrically actuated.

Thus, a device for injection of filling materials, particularly of highly viscous dermal injection material beneath a person's skin, is provided that does not impose on an operator, practitioner or user a requirement of exerting a significant amount of force for performing an injection. Simultaneously, due to the cable-free electrically actuated motor, the flexibility for the user, e.g. for moving around a patient, during the performance of an injection is in no manner restricted. Furthermore, the electrical actuation of the syringe guarantees a significantly higher degree of precision than pneumatic actuation. The control modes offered by the device guarantee a highly convenient and flexible operation of the device, its syringe cover provides for compatibility with numerous syringe sizes and types.

Other features and advantages of the present invention are mentioned in the dependent claims as well as in the description disclosing in the following, with reference to the figures, the invention in more detail.

In particular, the motor, the at least one battery and the gear box of the motor system are arranged, in the assembled state of the device, in non-linear manner with respect to each other, which allows for a reduced length as well as for an ergonomic shape of the device.

Furthermore, the device usually comprises a set of different syringe covers adapted to be mounted on the motor system, each syringe cover of said set of different syringe covers being adapted to house at least one, preferably several types, sizes, and shapes of syringes, and the set of syringe covers comprising different syringe covers designed such as to allow use of the device in combination with all different types, sizes, and shapes of syringes as well as all kind of injection materials. It is therefore advantageously possible to guarantee compatibility of the device with practically all syringes on the market.

According to one embodiment of the invention, a syringe cover is configured to accommodate at least parts of the syringe, the syringe cover having an opening at a distal end allowing for penetration of the syringe needle. By means of such an embodiment, the syringe can at least partially be hidden to the view of a patient. Furthermore, the syringe cover has a supporting function for moving and/or guiding the syringe in a desired way during operation. This also provides increased safety particularly for the case of application of high viscosity injection materials and/or low needle diameters, both leading to high pressures to be exerted.

According to a further embodiment, the syringe cover is provided with a cut-out section, for example designed as a window, allowing for observation of the syringe placed inside the syringe cover in an assembled state of the syringe cover with the motor system. Advantageously, this allows to an operator to observe and follow movements of the syringe parts, particularly of the syringe piston and gears, whereby the syringe can anyhow be hidden to the view of a patient, dependent on the design and location of the cut-out section serving as a window on the syringe cover.

According to a further embodiment, the syringe cover is configured to enable an exchange of a syringe needle of the syringe in an assembled state of the syringe cover with the motor system. This is for example useful, when during injection of material to a patient an exchange of syringe needles appears as recommendable and a longer interruption of the injection procedure shall be avoided. Particularly, this allows the operator to keep the syringe engaged in the device, to change the needle under operation, and to safely continue the operation.

According to a further embodiment, the syringe cover is configured to accommodate syringes of different sizes, the syringe cover playing the role of an adapter which is able to receive different syringe sizes and types, the adapter respectively syringe cover then being mounted onto the motor system with the syringe placed in its inside, shall it be with - or without its needle including the cap mounted on the latter. Thus, the flexibility for a user in acquiring and using any adequate syringe is increased.

According to a further embodiment, the syringe cover is provided with an end section configured to prevent an accidental expulsion of a syringe needle.

According to a further embodiment, locking means are provided to the syringe cover. Thereby, it is preferred that the locking means comprise locking verification means. The locking verification means may be provided, for example, as optical verification means such as an optical sensor or as a mechanical switch. With respect to this embodiment of the invention, it is further preferred that the locking verification means are configured to enable an operation of the injection device and an actuation of the motor system only if the syringe is correctly connected with the motor system. This increases the safety for a correct handling of the injection device.

According to a further, preferred, embodiment of the invention, the injection device is configured for manual control, i.e. activation and stopping of the device. This allows, due to the use of the operator's hands, for a more precise way of activation than, for example, by means of a foot pedal.

In particular, the syringe cover may be provided with several activation means for control of the motor system. This provides a high degree of comfort to an operator for an intuitive and flexible way of handling the injection device in the course of performing an injection.

Preferably, the motor system comprises an electronic command circuit equipped with a front activation control switch adapted to cooperate with said activation means of the syringe cover for activating the motor system depending on the user's operations on the syringe cover. This allows a particularly interesting and simple design of the syringe cover which thus primarily serves as a kind of lever actuating the front activation control switch following a corresponding movement of the operator's hand on the cover.

The electronic command circuit of the motor system may in a further embodiment also, or alternatively, comprise a rear activation control switch adapted to be operated directly by the user for activating the motor system. This allows the user to control the device, at his free choice, at the front or at the rear, or both.

In a further, preferred embodiment, it is even possible that the electronic command circuit of the motor system comprises means for choosing a location on the syringe cover and/or the motor system for activating the device. This further increases the flexibility for a user in the way of handling the injection device. Also, this is particularly advantageous for an equal comfort in device handling by both left-handed and right-handed persons.

Furthermore, the electronic command circuit of the motor system may comprise a control menu with input means for a menu-supported, user-controlled operation of the syringe. This allows to a user an easy, intuitive way of using the injection device. In particular, the user may program several parameters of the device like e.g. the injection speed of the material or the location on the syringe cover and/or the motor system for operating and activating the device.

Usually, an actuating force is transmitted from the first piston to the syringe by force transmission gears. Thereby, the gears are advantageously provided with diameters dependent on a requested torque and/or speed for driving the second piston of the syringe. Thus, it is enabled to configure the injection device specifically for the requirements on torque and speed associated with a certain kind of injection, e.g. for a specific injection material, a given syringe type, or a specific intervention on the patient to be treated.

According to a further embodiment, a gear is provided with a translucent casing and a marked section on its circumference for visualizing rotations of said gear. This allows to an operator to follow the number of rotations performed by the gear and thus, knowing the amount of fluid dispensed in the course of one rotation, to estimate the amount of injection material ejected from the syringe.

The attached figures exemplarily and schematically illustrate the principles as well as several embodiments of the present invention.
Figures 1 a and 1 b show exploded views from two different perspectives of a first embodiment of an injection device according to the invention.
Figure 2a shows in a perspective view a situation where the syringe is inserted into the syringe cover of the embodiment of figure 1a and figure 1b; figures 2b, 2c, 2d and 2e show different side views with the syringe cover of figure 2a; figure 2f illustrates another embodiment having a syringe cover that only encloses the posterior section of the syringe and the syringe barrel.
Figures 3a and3b further illustrate the locking mechanism for securing a syringe in a syringe cover, but also allowing a release of the syringe from the syringe cover in its assembled state with the motor system; figure 3c shows a further embodiment of a syringe cover allowing a release of the syringe from the syringe cover in its assembled state with the motor system, the distal part of the syringe cover comprising a lateral slot; figure 3d shows schematically in a lateral cross section a syringe held in the syringe cover according to figure 2f.
Figures 4a, 4b, 4c and 4d illustrate different modes of control of the inventive injection device for activation of an injection of material under the patient's skin.
Figures 5a and 5b illustrate the injection device in its assembled state with the pieces according to the previous figures, except for the embodiment of the syringe cover of figure 2f; figure 5b shows in more detail a control menu of an electronic command circuit of the motor system of the device.

In the following, the invention shall be described in detail with reference to the above mentioned figures.

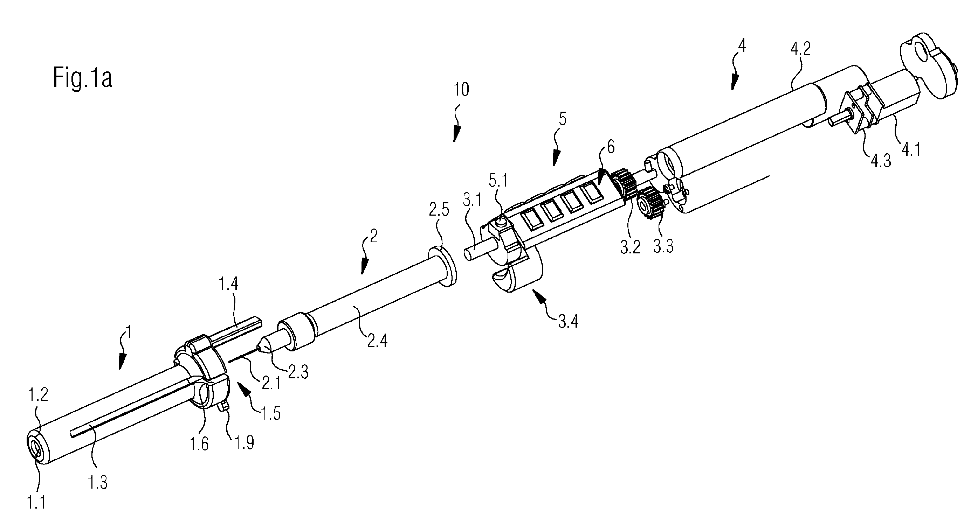

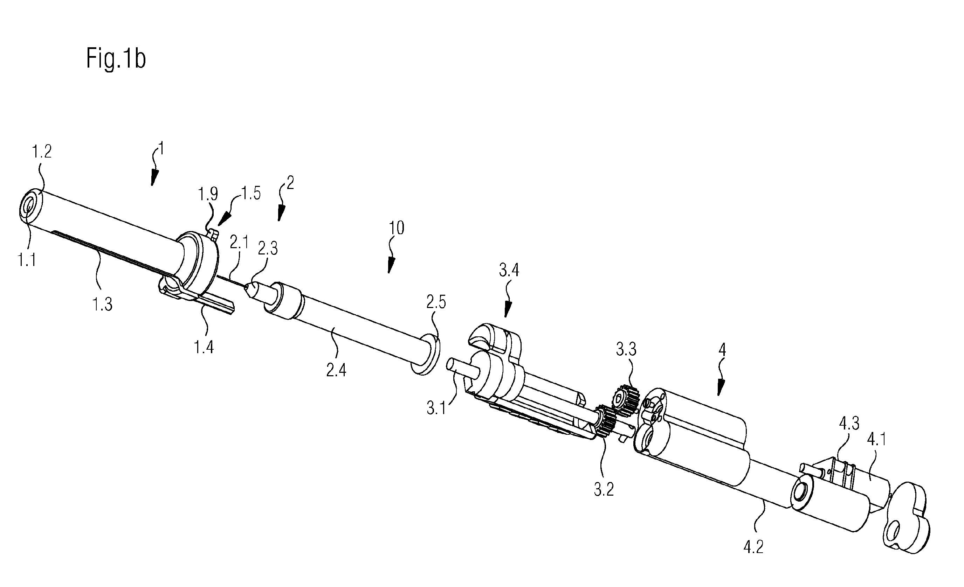

Figure 1a and figure 1b show exploded views from two different perspectives of a first embodiment of an injection device 10 according to the invention. A device such as proposed comprises two main units, which are a motor system 4 a syringe cover 1. The motor system 4 comprises a motor 4.1 electrically powered by at least one battery 4.2 and a gear box 4.3 with a first gear. The motor 4.1 may, for example, be provided as a brush or brushless motor or a stepper motor.

The electrical power source for the motor 4.1 may be at least one battery 4.2, which may be provided as an alkaline, a lithium or any other type of battery, for single use or rechargeable in nature. The power source preferably consists in a non-rechargeable battery of specific size and power specifically adapted for use in this device. This allows to realize a particularly simple solution of a cable-free operable device, with significantly less constraints both on the manufacturer's side, e.g. in terms of compliance with legal regulations in the medical sector concerning power sources and chargers, as well as on the user's side, e.g. in terms of keeping multiple pieces which otherwise would have to be furnished with the device, like a battery charger for rechargeable batteries. The power source may consist of a single cell unit or of multiple cell units working in series or in parallel. Alternatively, the power source may also be provided as a capacitor or super capacitor capable of storing electrical energy. The injection device according to the present invention thus does not require any cables outside its casing, i.e. it is a cable-free electrically actuated device.

Furthermore, in the present device, the motor 4.1, the at least one battery 4.2 and the gear box 4.3 of the motor system 4 are arranged, in its assembled state, in non-linear manner with respect to each other, particularly in a noticeably triangular manner, such as may be seen for example in figures 1 a and 1 b. In fact, contrary to prior art devices, the motor 4.1, the battery 4.2, and the gear 4.3 are not in line, but form a triangle motor-gear-battery. This allows to significantly shorten the total length of the device as well as to approach the centre of gravity of the motor system 4 relative to the syringe cover 1. This produces the further advantage, next to the shortened total length, that the devices becomes more easy to handle and has a more ergonomic shape, since its increased thickness and modified weight distribution is ideally adapted to be placed between the user's thumb and his other fingers, the whole device lying above the user's palm.

The gear box 4.3 is connected to the output shaft of the motor 4.1 and may be part of the same body or be a separate entity that cooperates with the motor 4.1 to increase the torque of the motor output shaft. Force transmission gears 3.2, 3.3 translate the rotational force of the gear box output shaft to a first piston or pressing thread 3.1 which itself acts on a second piston 2.2 mediated by an end section 2.5 of a syringe 2, the latter being housed in the syringe cover 1. The first gear 3.3 is rotationally fixed to the output shaft of the gear box 4.3 in a torque proof manner. The second gear 3.2 has a thread that matches the pressing thread 3.1 and is mechanically connected to said pressing thread 3.1. The gears can be provided with different diameters dependent on desired decreased or increased torque and speed of the system. The rotational speed of the gears is directly correlated with the advancement of the first piston or pressing thread 3.1 which is mounted such as to be rotatable inside the housing of the casing of the motor system 4, e.g. as a worm screw, such that the linear speed of advancement and force of the pressing thread 3.1 directly depends on the rotational speed and torque of the gears, in particular of the second gear 3.2.

Therefore, according to one embodiment, one of the gears 3.2, 3.3 could be used as a visual reference for allowing an operator to observe the speed and amount of injection material injection. Such visual reference of the advancement could be achieved by a translucent casing provided to said gear and a marked section on the circumference of the gear, so that a full rotation, corresponding to a known amount of injected material, could be clearly observed by the operator.

According to one embodiment of the invention shown in figure 1a, the piston or pressing thread 3.1 is partially surrounded by parts forming an electronic command circuit 5. The latter is provided with a control menu 6 with input means for a user-controlled operation of the syringe 2, these parts being described in more detail in a later section. At the distal end of this section of the injection device 10, in direction towards syringe 2 and syringe cover 1, locking means 3.4 are provided for connection with locking means 1.5 of the syringe cover 1. The mechanism for connecting the locking means 1.5 and 3.4 may, for example, be based on a bayonet style lock, a thread lock, a clip with a release button, or other equivalent means.

According to a preferred embodiment of the invention, the locking means 1.5 comprise supplementary locking verification means 1.9 for verifying if the syringe cover 1 together with the syringe 2 has properly been installed and locked with the motor systems 4. The locking verification means 1.9 may be provided, for example, as optical verification means such as an optical sensor or as a mechanical switch. Preferably the locking verification means 1.9 are provided with a control mechanism to allow operation of the injection device including actuation of the motor system 4 only if the syringe cover 1 is correctly connected with the motor system 4.

According to a further embodiment, an indication notch 1.6 is provided in a section of the syringe cover 1 close to the position for interfacing with the motor system 4. This indication notch 1.6 may, for example constitute an elongation of the cut-out section 1.3 and is intended for providing to an operator a visual and/or manual control if the syringe 2 and syringe cover 1 have properly been installed on the motor system 4.

The syringe 2 to be housed inside the syringe cover 1 comprises a syringe barrel 2.4, having a proximal end 2.5, for receiving the injection material and housing the syringe piston 2.2 to be acted upon by means of the first piston or pressing thread 3.1 of the injection device 10, the syringe having a distal end section 2.3 and a needle 2.1. The syringe 2 not forming part of the injection device as such may have any shape, size, and type.

The syringe cover 1 also realizes the function of an adapter for the syringe 2, preferably designed for receiving syringes of different size and possibly originating from different manufacturers, and is provided, according to different embodiments of the invention, in various forms and with different functionalities such as will become clear in the following. In general, the injection device 10 according to the present invention comprises a set of different syringe covers 1, 1' which are adapted to be mounted on the motor system 4 and shall be delivered to the user in order to allow the latter to use the device 10 together with all different types, sizes, and shapes of syringes 2 as well as all kind of injection materials on the market. Especially, each syringe cover 1, 1' of said set of syringe covers 1, 1' is adapted to house at least one, preferably several types, sizes, and shapes of syringes 2, and, since not all syringes on the market may be made compatible with the device 10 by use of one single syringe cover 1, the set of syringe covers 1, 1' comprises different syringe covers 1, 1' which are designed such that the whole set of syringe covers 1, 1' allows to guarantee compatibility of all syringes on the market with the device 10. Of course, further syringe covers may be furnished to the user after purchase of the device in case other, non-compatible types of syringes were developed and put on the market at a later point in time.

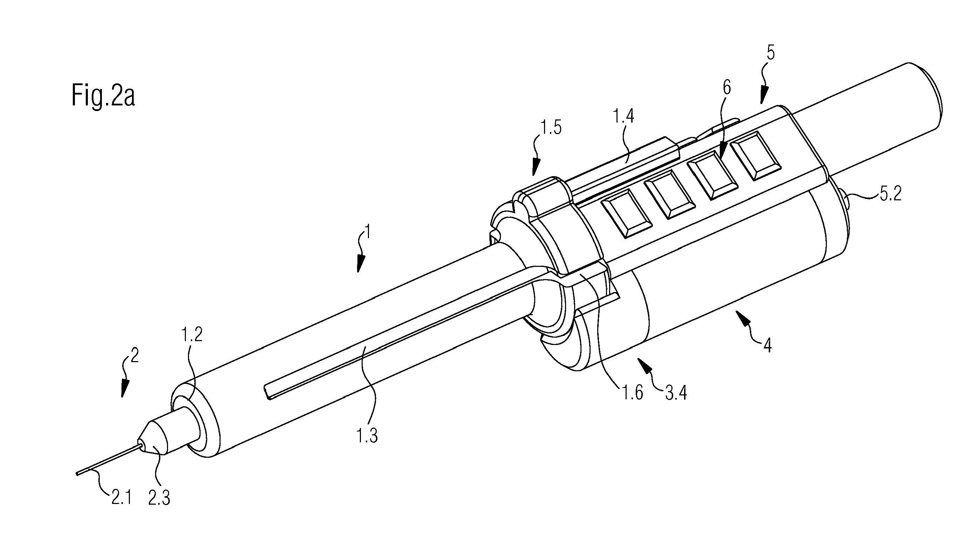

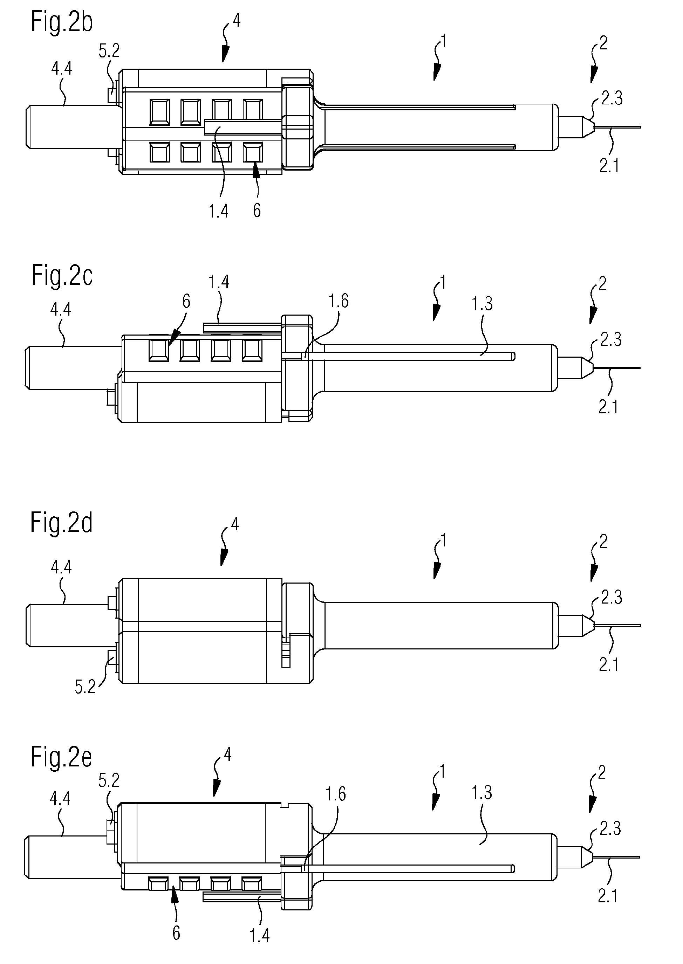

Typically, as visible for example at figure 2a or figures 2b to 2e, the syringe cover 1 has an opening 1.1 at its distal end 1.2 allowing for penetration of the syringe needle 2.1, together with the distal end section 2.3 of the syringe 2. The syringe cover 1 may be provided with a cut-out section or window 1.3 allowing an operator for observation of the syringe 2 inside the syringe cover 1 in its assembled state with the motor system 4. At its proximal end adapted to be mounted on the motor system 4, the syringe cover is provided with a lever 1.4 which serves as an operating lever for activating the device, such as will become clear in a later section of the present description.

Figure 2a shows in a perspective view a situation where a syringe 2 is inserted into the syringe cover 1, the distal end section 2.3 of the syringe 2 with needle 2.1 having been passed through the opening 1.1 at the distal end 1.2 of the syringe cover 1 of the embodiment of figure 1a and figure 1b. By means of a locking mechanism provided by connection of the locking means 1.5 of the syringe cover 1 and the locking means 3.4 of the motor system, the syringe 2 is connected to the motor system 4 and the first piston or pressing thread 3.1 of the injection device 10 may actuate the second piston 2.2 of the syringe 2.

According to this embodiment, the syringe cover 1 surrounds almost the entire syringe 2 and may hide it against the view of a patient. The entire enclosure of the syringe barrel 2.4 for receiving the injection material and housing the syringe piston 2.2 provides high safety, particularly for the case of application of high viscosity injection material, especially dermal fillers, and/or low needle diameters, both leading to high pressures to be exerted, such that accidental ejection of the whole or parts of the syringe 2 may be excluded due to the fact that the distal end section 2.3 of the syringe 2, i.e. the syringe needle 2.1, is held by a corresponding shoulder of the distal end 1.2 of the syringe cover 1. Figure 2b, figure 2c, figure 2d and figure 2e are different side views showing in detailed manner the syringe cover 1 of figure 2a, the parts of which have been described here above.

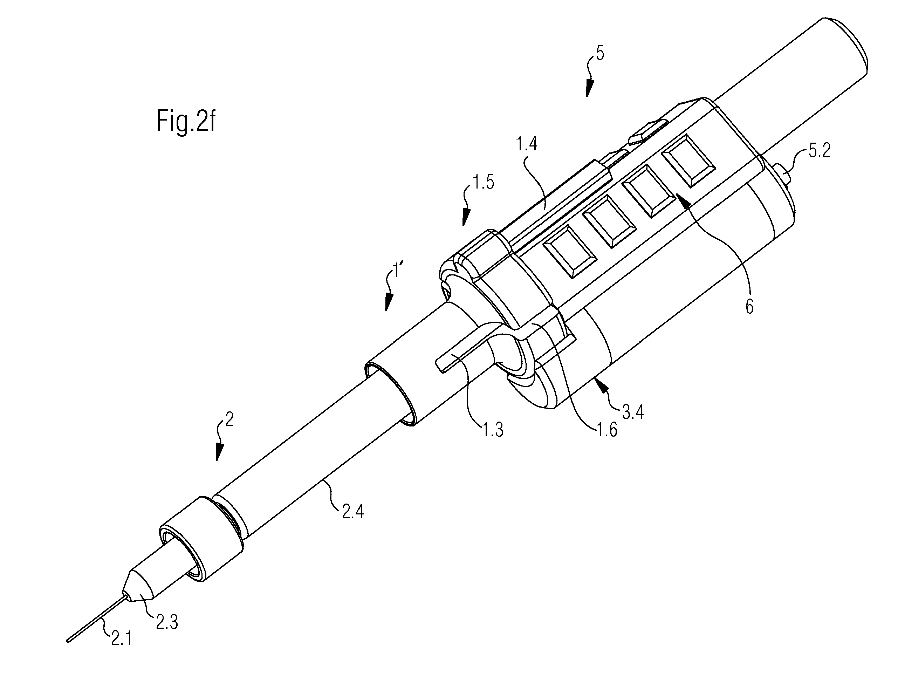

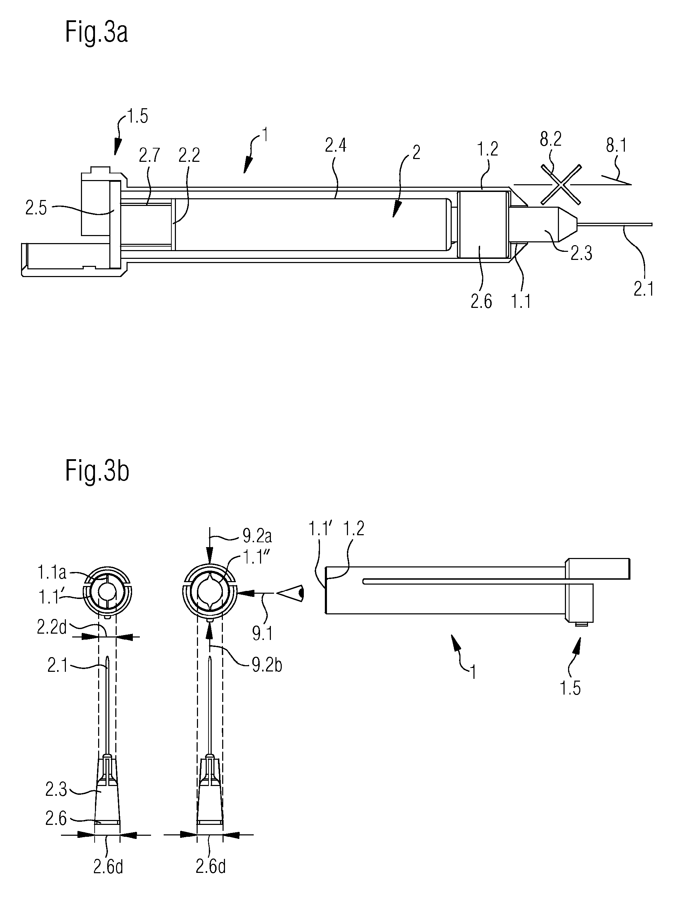

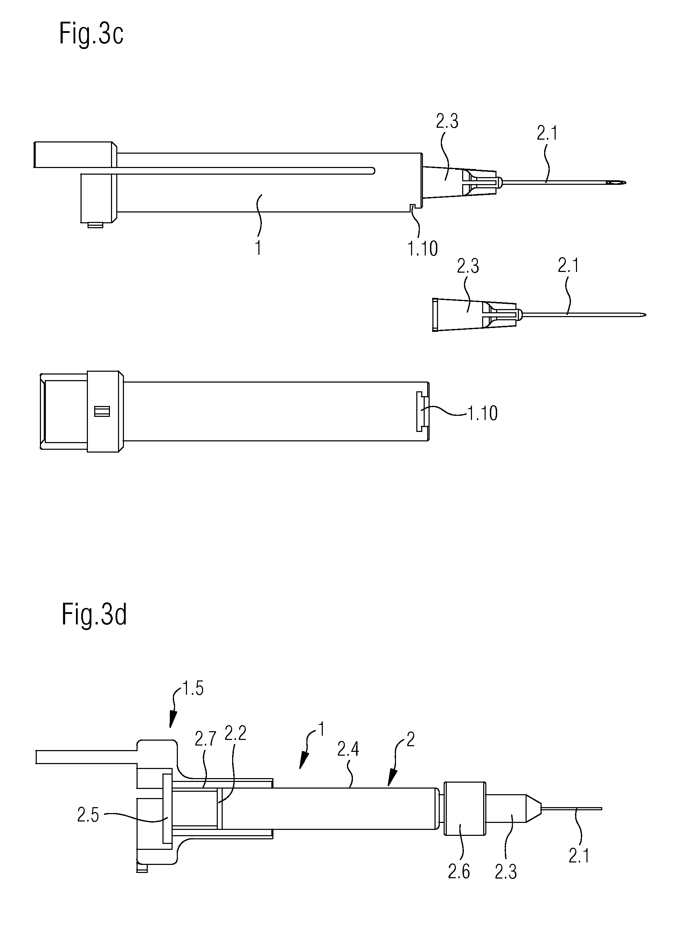

Figure 2f illustrates another, alternative, embodiment of a syringe cover 1' that only encloses the posterior section of the syringe 2 and the syringe barrel 2.4. In a way analogue to the embodiment of figure 2a, by means of a locking mechanism provided by connection of the locking means 1.5 of the syringe cover 1 and the locking means 3.4 of the motor system, the syringe 2 is connected to the motor system 4, and the first piston or pressing thread 3.1 of the device 10 actuates the second piston of the syringe 2, respectively. Although this alternative syringe cover 1' encloses only the posterior section of the syringe 2, respectively of the syringe barrel 2.4, accidental ejection of the whole or parts of the syringe 2 from the syringe cover 1, respectively from the device 10, in case of application of high pressures may be excluded by providing a corresponding shoulder at the proximal end of the syringe cover 1 cooperating with the proximal end 2.5 of the syringe 2, such as is visible e.g. in figure 3d. The syringe cover 1 shown in figures 2a to 2e may be equipped with similar means, such as visible schematically in figure 3a.

Both the syringe cover 1 and 1' may be intended for single use and be fabricated in an adequate disposable material. Alternatively, these syringe covers 1, 1' may be intended for multiple uses and accordingly be fabricated in a material (such as PEEK or PPSU) that can be sterilized without deformation. This material also represents an attractive option for the components of the locking mechanism, which may thus be provided in a relatively durable form, particularly adapted for the case of multiple use.

In general, the syringe covers 1, 1' are preferably dedicated to be provided packaged in a single use clean bag in order to guarantee not having been entered into contact with any external body or material prior to application by an operator or user.

Figure 3a and figure 3b further illustrate the locking mechanism for securing a syringe 2 and, in particular, the syringe needle 2.1 in a syringe cover 1 almost entirely surrounding the former, such as described above, but also allowing a release of the syringe needle 2.1 from the syringe cover 1 in an assembled state of the syringe cover 1 with the motor system 4. Several alternative solutions may be provided for this effect, one of it being illustrated at figure 3b.

Figure 3a shows schematically in a lateral cross section a syringe 2 held in the syringe cover 1 according to figures 2a to 2e, the syringe 2 comprising a head section 2.7 of the syringe piston 2.2, for pressing material out of the syringe barrel 2.4, at its proximal end and a luer lock 2.6 close to its distal end 2.3. The volume inside the syringe barrel 2.4 comprises some injection material. The arrow 8.1 indicates a force driving the syringe 2 towards the end section 1.2 of the syringe cover 1, and cross 8.2 indicates the prevention of an expulsion of the syringe needle 2.1 due to the design of the syringe cover 1 according to this embodiment, such as already described above.

Figure 3b illustrates in different views an embodiment of a syringe cover 1 where the opening at the distal end 1.2 of the syringe cover 1 is provided as a deformable opening 1.1' provided with a slit 1.1 a, thus allowing to deform the circular opening 1.1' under exposure of squeezing forces 9.2a. 9.2b, e.g. exerted by manual pressure on the lateral sides of the syringe cover 1. This allows to deform the syringe cover 1 from showing a merely circular opening to adapting an ellipsoidal opening 1.1 ", such as illustrated schematically in figure 3b. Thus, the diameter of the opening 1.1' of the syringe cover 1 does not allow, in the not squeezed state, a penetration and pass of the syringe needle 2.1, respectively of the corresponding distal end section 2.3 of the syringe 2 comprising the syringe needle 2.1, further outwards the circular opening 1.1' due to the luer lock 2.6. However, once the diameter of the syringe cover opening 1.1' being deformed to a geometrical shape 1.1 ", pushing the syringe needle 2.1, respectively of the corresponding distal end section 2.3 of the syringe 2 comprising the syringe needle 2.1, becomes possible in view of the diameter 2.6d of the luer lock 2.6 as compared to the deformed opening 1.1 ". It should be noted that the squeezing forces 9.2a. 9.2b producing the required deformation of the syringe cover 1 are perpendicular to the forces produced by the pressure of the piston 3.1, such that the latter may not provoke accidental expulsion of the syringe needle 2.1.

According to another embodiment not illustrated in the figures, at least the distal part of the syringe cover 1, in direction of the needle 2.1, is fabricated in an elastic material that can be deformed, under exertion of pressure forces to a geometrical shape allowing for extracting the syringe needle 2.1 from the syringe cover 1.

According to another embodiment illustrated in the figure 3c, the distal part 1.2 of the syringe cover 1 comprises a lateral slot 1.10 of a width sensibly similar, but slightly less than the width of the distal end section 2.3 of the syringe 2 comprising the syringe needle 2.1. The latter therefore may be introduced in the slot 1.10 of the syringe cover 1 by clipsing the distal end section 2.3 laterally onto the syringe cover 1, with the cover being mounted on the motor system 4 and containing a syringe 2. Therefore, also this embodiment allows mounting and releasing of the syringe needle 2.1 on the syringe 2 in an assembled state of the syringe cover 1 with the motor system 4.

Also, many legislations provide for medical regulations requiring for reasons of security a needle cap not illustrated in the figures to be released from the syringe needle 2.1 only at the moment when the medical intervention effectively starts. Another embodiment of a syringe cover 1 not illustrated in the figures thus may comprise at the distal end 1.2 of the syringe cover 1 simply an opening 1.1 corresponding to the largest of the diameters of the distal end section 2.3 of the syringe 2 comprising the syringe needle 2.1 and of said needle cap, such that the syringe needle 2.1 together with its needle cap may be mounted on and released from the syringe 2 in any case and at any time, the syringe cover 1 with the syringe 2 in its inside being in an assembled state with the motor system 4 or not. This guarantees that the syringe needle 2.1 does neither touch the cover 1 nor risks to hurt the practitioner or the patient before the beginning of the treatment.

In another embodiment of a syringe cover 1 not illustrated in the figures, such a cover may moreover be provided at its distal end 1.2 with a thread matching the tread usually formed at the distal end section 2.3 of the syringe 2 comprising the syringe needle 2.1, such that both parts 1.2, 2.3 may be mounted together in secure manner.

Therefore, with the embodiments of a syringe cover 1 according to figures 2a to 2e, respectively 3a, 3b, and 3c, the syringe needle 2.1, respectively the corresponding distal end section 2.3 of the syringe 2 comprising the syringe needle 2.1, may be removed by an operator whilst leaving the syringe main body and the syringe cover 1 in place on the motor system 4. This allows the operator to keep the syringe engaged in the device 10, to change the needle 2.1 during operation, and to safely continue the intervention in course.

Figure 3d shows schematically in a lateral cross section a syringe 2 held in the syringe cover 1' according to figure 2f. In case of this embodiment of the syringe cover, the syringe 2 is fastened by the locking means 1.5 of the syringe cover 1' to the motor system 4 and/or its supplements.

Following the above description of the mechanical parts of an injection device 10 according to the present invention, the control of the device shall now be described in more detail. In general, an injection device 10 according to the present invention is configured for manual activation, i.e. for control by the hands of the user or practitioner to allow a high precision control. This is in consistency with the approach of a battery driven, cable-free motor system 4 of the device.

Furthermore, the motor system 4 comprises an electronic command circuit 5 equipped with a front activation control switch 5.1 adapted to cooperate with activation means 1.4, 1.4', 1.7, 1.8 of the syringe cover 1, 1' for activating the motor system 4 depending on the user's operations on the syringe cover 1, 1'. The electronic command circuit 5 of the motor system 4 also comprises a rear activation control switch 5.2 to be operated directly by the user for activating the motor system 4. As will become clear in the following the syringe cover 1, 1' is mounted on the motor system 4 of the device 10, in the assembled state of these pieces, in such a manner that the lever 1.4 of the syringe cover 1, 1' comes to lie on top of the front activation control switch 5.1, without activating the switch 5.1 in its normal position, respectively during normal operation of the device. Also, the syringe cover 1, 1' is mounted on the motor system 4 of the device 10, in the assembled state of these pieces, in such a manner that it is slightly movable, e.g. in a sensibly rotational manner at its proximal end mounted on the motor system 4, allowing the lever 1.4 of the syringe cover 1, 1' to activate said front activation control switch 5.1 once the operator or practitioner performs one of the described actions on the syringe cover 1, 1' during operation of the device, in order to activate or stop operation of the injection device 10.

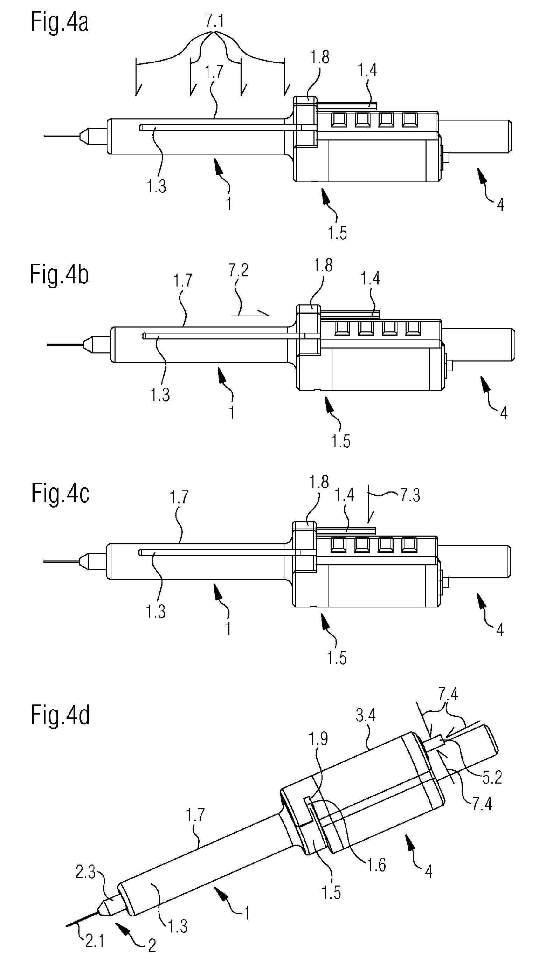

Figure 4a, figure 4b, figure 4c and figure 4d illustrate schematically different actions that may be performed by the operator or practitioner, respectively different modes of control, i.e. of activation, respectively of stopping, of the inventive injection device for activation or stopping of an injection of material under the patient's skin by manual activation of the device. In fact, the device is activated or stopped using the above mentioned, different activation control means 5.1, 5.2 which may be controlled indirectly by use of the syringe cover 1 or directly on the motor system 4. Figure 4a, figure 4b and figure 4c are identical except for the indication of the activation mode, performed in the cases illustrated on these figures via the syringe cover 1.

Figure 4a illustrates a first control mode of the injection device 10 where a lateral surface 1.7 of the syringe cover 1 may be used by the operator to apply a small pressure 7.1 oriented sensibly perpendicular to the longitudinal axis of the syringe cover 1 for activating or stopping the motor 4.1 of the motor system 4 of the device 10, this by simply and slightly pushing down the syringe cover 1 of the injection device 10. In fact, each such pressure 7.1 on the syringe cover 1 provokes, by a small movement of the syringe cover 1 with respect to the motor system 4 onto which it is mounted, activation of said front activation control switch 5.1, which in turn activates or stops the motor 4.1 via said electronic command circuit 5. A first push activates injection of injection material, a release or second push stops the injection. This control mode is particularly beneficial for operators who prefer controlling the injection device with the index or middle finger of their hands, allowing a precise and easy use of the injection device by users with different size hands.

Figure 4b illustrates a second control mode of the injection device 10 where a section 1.8 of the lever 1.4 of the syringe cover 1 may be used by the operator to apply a little pressure 7.2 oriented sensibly parallel to the longitudinal axis of the syringe cover 1, in the direction of the motor system 4, for activating or stopping the motor 4.1 of the motor system 4 of the device 10. Again, each such pressure 7.2 on the syringe cover 1 provokes, by a small movement of the syringe cover 1, respectively of its lever 1.4, with respect to the motor system 4, activation of said front activation control switch 5.1, which in turn activates or stops the motor 4.1. This control mode is particularly beneficial for operators who prefer controlling the injection device with the thumb of their hands, but may of course be operated by using other fingers or parts of the hand, depending on the hand size of the operator or practitioner. Thus, also this mode allows a precise, easy, and flexible use of the injection device by users.

Figure 4c illustrates a third control mode of the injection device 10 where the lever 1.4 of the syringe cover 1 may be used by the operator to apply a little pressure 7.3 oriented sensibly perpendicular to the longitudinal axis of the syringe cover 1 for activating or stopping the motor 4.1 of the motor system 4 of the device 10, this by simply pushing down the lever 1.4 towards the main body of the injection device 10 onto which the syringe cover 1 is mounted. Again, each such pressure 7.3 provokes, by a small movement of the syringe cover 1, respectively of its lever 1.4, with respect to the motor system 4, activation of said front activation control switch 5.1, which in turn activates or stops the motor 4.1. Also this control mode is particularly beneficial for operators who prefer controlling the injection device with the thumb of their hands, but may be operated by using other fingers or parts of the hand, depending on the hand size of the operator or practitioner, thus allowing a precise, easy, and flexible use of the injection device.

Figure 4d illustrates a slightly different, fourth control mode of the injection device 10 allowing manual activation of activation control means directly on the motor system 4, instead of indirectly activating activation control means by use of the syringe cover 1 such as realized in the first three activation modes. In fact, the fourth control mode allows using a rear activation control switch 5.2 which is situated on the rear area of the motor system 4 and may be pressed from different directions 7.4 in order to activate or stop the motor 4.1 of the motor system 4 of the device 10. This control mode is particularly beneficial for operators who prefer controlling the injection device with the thumb of their hands. Depending on the manner in which the operator or practitioner prefers to hold the injection device 10, the rear activation control switch 5.2 may, however, also be activated by using other fingers or parts of the hand, thus allowing a precise, easy, and flexible use of the injection device.

The control modes as illustrated in these figures 4a to 4d allow a user to select, depending on his personal preferences as well as on the necessities during the treatment of a patient, where and by which manual action the injection device shall be activated. This is particularly advantageous in the course of certain treatments of aesthetic medicine where the user should be provided with the highest possible flexibility in injection device operation.

For all control modes of the injection device 10, the electronic command circuit 5 of the motor system 4 may be programmed such as to allow control of the device 10 by a first and second push which activate, respectively stop the motor 4.1 of the motor system 4, by a first push followed by release of said push to activate, respectively stop the motor 4.1, or by one of these modes at the user's choice according to his personal preferences. For many treatments, the control mode allowing to activate, respectively stop the motor 4.1 by a first push followed by release of said push is a preferred embodiment of the control of the injection device 10.

The electronic command circuit 5 of the motor system 4 preferably also comprises means for choosing a location on the syringe cover 1, 1' and/or the motor system 4 for controlling, i.e. for activating or stopping, the device, e.g. by comprising a corresponding menu switch. Moreover, in an even more preferred embodiment, the device also offers other parameters of the injection of an injection material during use of the injection device may be pre-selected before activating the injection.

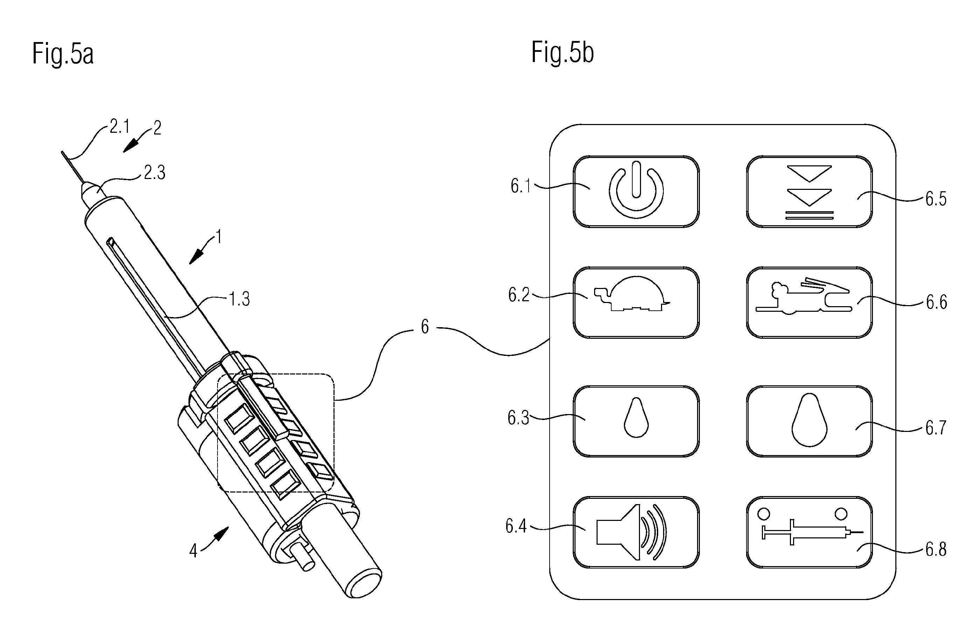

Figure 5a illustrate, as an example, the injection device 10 according to the previous figures, except for the embodiment of the syringe cover 1' of figure 2f, in its assembled state. Of course, the injection device 10 can also be combined with the embodiment of the syringe cover 1' of figure 2f. Figure 5b shows in schematic manner an enlarged view of a control menu 6 of the electronic command circuit 5, the control menu 6 comprising input keys indicated by adequate symbols for an operator or practitioner. The symbols shown in the figures are only examples and may, of course, be modified such as to indicate the corresponding function of the injection device in other manner. The symbols may be illuminated, too.

Figure 5b shows an arbitrary selection of symbols for input keys placed on the motor system 4 of the injection device 10 and allowing an operator to perform corresponding actions, respectively to choose corresponding functions or preselect some parameters of the injection of an injection material during use of the injection device on the electronic command circuit 5, respectively the device 10:
6.1: Activation or deactivation of the electronic command circuit, i.e. the device 10;
6.2: "Turtle mode", i.e. slow injection, which may e.g. correspond to < 30 mm/min piston advancement;
6.3: "Small drop mode", i.e. injection of small fluid amounts per injection step;
6.4: "Loudspeaker", i.e. activation or deactivation of sound, e.g. of an acoustic reference like beep in intervals, indicating if the injection device is delivering fluid or not;
6.5: "Reverse mode", i.e. command for moving the piston back to its start position once the syringe 2 is empty or the treatment finished, thereby releasing the syringe;
6.6: "Rabbit mode", i.e. fast injection, which may e.g. correspond to > 50 mm/min piston advancement;
6.7: "Large drop mode", i.e. injection of large fluid amounts per injection step;
6.8: "Choice of activation mode", allowing the user to select amongst one or several of the four activation modes described above, thus to select where the injection device shall be activated, i.e. at the front or the back of the injection device 10 or at both locations.

The above mentioned functions or possibility of pre-selection of parameters of the injection of an injection material during use of the injection device may, in some simplified embodiments of an injection device according to the present invention, not all be present on the device. The same applies analogously to the realization of the above described control modes, respectively the corresponding activation control means. In general, it is pointed out that all embodiments of the invention described above, also with respect to the figures, may be combined with one another if not explicitly stated otherwise.

The electronic command circuit 5 of the motor system 4 may also be programmed such as to automatically recognize the syringe cover 1, 1' which is currently mounted on the motor system 4. To that end, each syringe cover 1, 1' of the set of different syringe covers 1, 1' needs to be equipped, e.g. at the surface of the proximal end oriented towards the motor system 4 or the lower surface of the lever 1.4 or the like, with a pattern or other adequate means allowing recognition of specific syringe cover 1, 1' amongst the set of syringe covers 1, 1'. Correspondingly, the motor system 4 is in that case equipped with identification means allowing to read the pattern. This allows in advantageous manner, for example, to have automatically chosen by the electronic command circuit 5 an injection mode specifically adapted for the syringe 2 typically mounted in the given, identified syringe cover 1, 1', respectively for the product usually contained in that syringe 2. In case the identified syringe cover 1, 1' exceptionally contained another syringe as compared to the one typically mounted, the parameters of injection of the device 10 may, of course, be manually adapted by use of the above described control menu 6.

In light of the above description of the structure and of the operating mode of the present invention, its advantages are clear. Primarily, an improved injection device for application in the area of aesthetic medicine, for administering injection material is provided, the device being very comfortable to handle by an operator, especially in manner similar to a use of a pen. The device is provided in a compact, low-weight configuration may be fabricated at a total weight of about 60 g, and does not require cumbersome cable connections due to its cable-free construction principle, all of which increases the advantages of the proposed device specifically adapted for pen-like use. The motor-assisted injection of injection material allows the practitioner of aesthetic medicine to concentrate his effort on the aesthetic treatment due to the absence of manual efforts on his side, in any case the required forces for operating the device are in or even below the range of 50 N. Simultaneously, the injection device is easily and intuitively operable, without requiring an expert installation at the application site. The various control respectively activation modes allow a particularly convenient and flexible handling of the injection device according to the personal preferences of the practitioner or the necessities of a given treatment to be performed. The syringe cover allows for use of many different sizes and types of syringes originating from various manufacturers and allows to disguise the syringe from the patient which is an often underestimated psychological factor.

## Claims

1. Injection device (10) for aesthetic medicine, for administering a filling material beneath the skin of a person by means of a syringe (2), the injection device (10) comprising
- a motor system (4) comprising a motor (4.1) configured to drive a first piston (3.1), and
- at least a syringe cover (1, 1') adapted to be mounted on the motor system (4) and to accommodate a syringe (2) comprising at least a syringe barrel (2.4) housing the filling material, a syringe needle (2.1) and a second piston (2.2) located in the syringe and drivable by the first piston (3.1) for ejecting a quantity of filling material out of the syringe (2),
**characterised by the fact that** the motor system (4) is cable-free electrically actuated.

2. Injection device (10) according to the preceding claim, **characterised by the fact that** the motor (4.1), the at least one battery (4.2) and the gear box (4.3) of the motor system (4) are arranged, in the assembled state of the device (10), in a non-linear manner with respect to each other.

3. Injection device (10) according to one of the preceding claims, **characterised by the fact that** the device (10) comprises a set of different syringe covers (1, 1') adapted to be mounted on the motor system (4), each syringe cover (1, 1') of said set of different syringe covers (1, 1') being adapted to house at least one, preferably several types, sizes, and shapes of syringes 2, and the set of syringe covers (1, 1') comprising different syringe covers (1, 1') designed such as to allow use of the device (10) in combination with all different types, sizes, and shapes of syringes (2) as well as all kind of injection materials.

4. Injection device (10) according to one of the preceding claims, **characterised by the fact that** each syringe cover (1, 1') is configured to accommodate at least parts of the syringe (2), the syringe cover (1, 1') having an opening (1.1) at a distal end (1.2) allowing for penetration of the syringe needle (2.1).

5. Injection device (10) according to one of the preceding claims, **characterised by the fact that** the syringe cover (1, 1') is configured to enable an exchange of a syringe needle (2.1) of the syringe (2) in an assembled state of the syringe cover (1, 1') with the motor system (4).

6. Injection device (10) according to one of the preceding claims, **characterised by the fact that** the syringe cover (1) is provided with a distal end (1.2) configured to prevent an accidental expulsion of a syringe needle.

7. Injection device (10) according to one of the preceding claims, **characterised by the fact that** the syringe cover (1, 1') is provided with locking means (1.5).

8. Injection device (10) according to the preceding claim 7, **characterised by the fact that** the locking means (1.5) comprise locking verification means (1.9) which are configured to enable an operation of the injection device (10) and an actuation of the motor system (4) only if the syringe cover (1) is correctly mounted on the motor system (4).

9. Injection device (10) according to one of the preceding claims, **characterised by the fact** that it is configured for manual control.

10. Injection device (10) according to one of the preceding claims, **characterised by the fact that** the syringe cover (1, 1') is provided with activation means (1.4, 1.4', 1.7, 1.8) for controlling the motor system (4).

11. Injection device (10) according to the preceding claim 10, **characterised by the fact that** the motor system (4) comprises an electronic command circuit (5) equipped with a front activation control switch (5.1) adapted to cooperate with said activation means (1.4, 1.4', 1.7, 1.8) of the syringe cover (1, 1') for controlling the motor system (4) depending on the user's operations on the syringe cover (1, 1').

12. Injection device (10) according to one of the preceding claims 9 to 11, **characterised by the fact that** the electronic command circuit (5) of the motor system (4) comprises a rear activation control switch (5.2) adapted to be operated directly by the user for controlling the motor system (4).

13. Injection device (10) according to one of the preceding claims 11 to 12, **characterised by the fact that** the electronic command circuit (5) of the motor system (4) comprises means for choosing a location on the syringe cover (1, 1') and/or the motor system (4) for controlling the device.

14. Injection device (10) according to one of the preceding claims 11 to 13, **characterised by the fact that** the electronic command circuit (5) of the motor system (4) comprises a control menu (6) with input means (6.1, 6.2, 6.3, 6.4, 6.5, 6.6, 6.6, 6.7, 6.8) for a menu-supported, user-controlled operation of the syringe (2).

15. Injection device (10) according to one of the preceding claims 11 to 14, **characterised by the fact that** the electronic command circuit (5) of the motor system (4) comprises identification means allowing to automatically recognize the syringe cover (1, 1') amongst the set of syringe covers (1, 1') currently mounted on the motor system (4) and to automatically select adequate parameters of injection.
